# FASCICULE DE BREVET EUROPEEN

(11) **EP 2 719 292 B1**
(45) Date de publication et mention de la délivrance du brevet: **04.01.2017**
(21) Numéro de dépôt: 13306393.3
(22) Date de dépôt: 09.10.2013
(51) Int. Cl.: A23L 33/17, A61K 35/20

(54) **PRODUIT DE NUTRITION MÉDICALE DESTINÉ À ÊTRE ADMINISTRÉ AUX PERSONNES OBÈSES RÉCEMMENT OPÉRÉES EN CHIRURGIE BARIATRIQUE**
MEDIZINISCHES NAHRUNGSMITTEL ZUR VERABREICHUNG AN ÜBERGEWICHTIGE PERSONEN, BEI DENEN VOR KURZEM EIN BARIATRISCH-CHIRURGISCHER EINGRIFF VORGENOMMEN WURDE
MEDICAL NUTRITION PRODUCT INTENDED FOR BEING ADMINISTERED TO OBESE PERSONS RECENTLY OPERATED IN BARIATRIC SURGERY

(30) Priorité: 09.10.2012 FR 1259607
(43) Date de publication de la demande: 16.04.2014
(73) Titulaire: International Nutrition Research Company, 1130 Luxembourg (LU)
(72) Inventeur: Vincent, Claude, 33000 Bordeaux (FR)
(74) Mandataire: Aquinov

(56) Documents cités:
- WO-A1-2009/153065
- WO-A1-2010/114627
- WO-A2-2010/043415
- FR-A1- 2 981 544
- FR-A1- 2 981 545

## Description

La présente invention concerne un produit de nutrition médicale spécifiquement destiné aux personnes obèses récemment opérées en chirurgie bariatrique. Cette dernière décennie, le nombre d'individus obèses n'a cessé d'augmenter, notamment dans les pays développés, avec surtout une forte croissance du nombre de personnes présentant une obésité sévère ou morbide.

La restriction calorique et le rééquilibrage alimentaire sont impuissants à normaliser le poids de ces personnes qui perdent quelques dizaines de kilogrammes et les reprennent sur la durée. Or, les risques entraînés par l'obésité sont nombreux en particulier pour l'obésité abdominale. En effet, on sait que l'obésité abdominale augmente le risque cardiométabolique, qui désigne la présence chez un individu de plusieurs signes cliniques et biologiques qui accroissent le risque de maladies cardiaques, d'accidents cardiovasculaires et de diabète de type 2.

Devant cette menace, les médecins ont de plus en plus recours à la chirurgie bariatrique, chirurgie consistant à restreindre la quantité et l'absorption des aliments. Elle englobe à la fois :
- la chirurgie restrictive qui diminue la taille de l'estomac et donc les quantités ingérées : anneau gastrique, gastrectomie longitudinale ou sleeve ; et
- la chirurgie malabsorptive et restrictive qui permet de réduire à la fois la quantité d'aliments et leur absorption : by-pass et dérivation pancréatique. Le recours à ce type d'interventions permet d'aider les patients opérés à perdre du poids de façon très importante de l'ordre de 2 à 4kg par semaine pour aller jusqu'à une perte atteignant de 30 à 40% du poids initial.

De plus, en théorie, l'opération permet également de guérir le diabète et de réduire significativement le risque cardiométabolique.

Toutefois, malgré les effets positifs procurés par la chirurgie, il s'avère que le taux d'échec dans le long terme est très important. Cet échec provient rarement de la technique chirurgicale, mais est imputable le plus souvent à l'absence de solution nutritionnelle adaptée, y compris au cours des jours et des semaines qui suivent l'opération.

Après l'intervention, le patient est mis en jeune durant 2 jours, ce qui provoque un état de cétose important dans ses conséquences pour les premiers jours d'alimentation. Ensuite, pendant 1 mois la personne doit absorber des aliments sous forme liquide ou semi-liquide en 5 à 6 prises par jour, cette alimentation fractionnée étant obligatoire en raison de la nouvelle taille de son estomac de 30 à 300 ml en moyenne. Puis, petit à petit, selon ses possibilités biologiques, le patient s'alimente sous forme plus solide pour arriver à une alimentation normale. Habituellement, l'absorption d'énergie est de 700 kcal pour les premières semaines pour monter progressivement à 1300 kcal et se recaler sur sa nouvelle Dépense Energétique Totale. Durant cette période d'adaptation, la personne fait souvent de nombreuses erreurs nutritionnelles comme s'alimenter avec des sucres rapides à charges glycémiques élevées ou même avec des aliments à charge calorique importante. Elle reprend souvent ses habitudes antérieures considérant que l'intervention chirurgicale a traité définitivement son problème, ce qui n'est pas le cas.

Actuellement, il n'existe aucune solution diététique efficace et adaptée aux besoins de ces personnes venant de subir une intervention de chirurgie bariatrique. Les solutions existantes sont des préparations domestiques selon les indications du diététicien du service chirurgical ou des produits diététiques généraux qui ne sont pas adaptés aux caractéristiques médicales du patient obèse, opéré et devant restreindre quantitativement son alimentation à cause de la nouvelle taille de son estomac.

Il subsiste donc un besoin pour une solution adaptée pour ce type de personne qui à la fois conserve certaines caractéristiques des obèses, en particulier les déficiences ou l'état inflammatoire ou encore le stress chronique (auquel s'ajoute le stress de l'opération) qui a subit les conséquences digestives et métaboliques d'une opération en particulier dans le cadre de la chirurgie malabsorptive, et qui a des contraintes de restrictions quantitatives très importantes avec une perturbation alimentaire majeure.

WO2010/043415 A2 décrit une composition nutritionnelle comprenant du lactosérum.

L'objectif de la présente invention est donc de répondre à ce besoin et de proposer une solution efficace, scientifique, standardisée, naturelle et facile d'utilisation, adaptée aux caractéristiques médicales du patient obèse, récemment opéré et présentant un estomac de petite taille, ne pouvant supporter que des aliments facilement digérables et ayant une osmolarité particulière.

A cet effet, l'invention propose d'utiliser une composition diététique particulière, comprenant au moins les constituants suivants :
- un isolat de lactosérum, et
- un concentrat de lactosérum.

Avantageusement, une telle composition peut être utilisée comme produit de nutrition médicale à administration orale destiné à améliorer l'état médical d'une personne obèse récemment opérée, en phase post opératoire à court terme d'une intervention de chirurgie bariatrique.

Cette composition peut être utilisée pour remplacer et/ou complémenter l'alimentation de la personne obèse récemment opérée.

L'invention est à présent décrite en détails.

L'invention vise donc une composition diététique pour son utilisation comme produit de nutrition médicale à administration orale destiné à améliorer l'état médical d'une personne obèse récemment opérée, en phase post opératoire d'une intervention de chirurgie bariatrique, comprenant au moins les constituants suivants :
- un isolat de lactosérum, et
- un concentrat de lactosérum.

Par composition diététique ou produit diététique au sens de l'invention, on entend un produit destiné à une alimentation particulière, comme seule alimentation, c'est-à-dire comme substitut de repas, ou en complément d'un régime restrictif ou d'une alimentation équilibrée.

Par produit de nutrition médicale au sens de l'invention, on entend un produit de santé particulier à savoir un produit diététique ou produit de nutrition médicale destiné à des fins médicales spéciales.

Par « phase post-opératoire à court terme » ou « récemment opéré » au sens de l'invention, on entend une période d'un mois qui suit immédiatement l'opération. Une personne obèse récemment opérée, en phase post opératoire d'une intervention de chirurgie bariatrique, présente des caractéristiques particulières liées à son état :
- d'obèse avec des déficiences (notamment tryptophane, zinc, magnésium, vitamine B6, vitamine D) et des excès (notamment cortisol et inflammation de bas grade qui retarde la cicatrisation)
- de nouvel opéré qui doit s'adapter à un estomac dont la taille a été réduite de plus de 90% avec la nécessité de besoins spécifiques en macronutriments (en particulier protéines plus digestes) et micronutriments liés non seulement à son état d'obèse mais également à la perte de poids rapide et à la création de carences du fait de la diminution de la taille de l'estomac.

Par isolat de lactosérum au sens de l'invention on entend un extrait de lactosérum qui contient moins de 1% de lactose et de matières grasses.

Par concentrat de lactosérum au sens de l'invention on entend un extrait de lactosérum obtenu par concentration d'un lactosérum.

La composition ou le produit diététique selon l'invention est particulièrement adaptée aux personnes obèses ayant subi une opération de chirurgie bariatrique, en phase post opératoire à court terme. Elle permet à ces personnes de passer efficacement cette période post opération bariatrique en limitant les risques opératoires et en ayant une meilleure récupération tout se préparant à éviter des états pathogènes graves qui pourraient subvenir ultérieurement.

En effet, une telle composition est capable d'agir à la fois sur les problématiques liées au statut d'obèse de la personne, sur celles engendrées par l'intervention bariatrique qu'elle a subi et sur celles liées à la taille réduite de son estomac. Tout d'abord, la personne obèse souffre d'une déficience importante en tryptophane provoquée soit par le catabolisme des adipocytokines inflammatoires, en particulier l'adipocytokine TNFa, sécrétée par le macrophage du tissu adipeux viscéral, soit par le cortisol dont la sécrétion est commandée par le stress chronique qui dérègle l'axe neurovégétatif. La conséquence de ce déficit en tryptophane circulant est que ce dernier ne peut pas passer la barrière encéphalique pour synthétiser la sérotonine car sa concentration par rapport aux acides aminés neutres (avec qui il est en compétition dans le transport) est trop faible. Sur le plan physiologique, la déficience en sérotonine cérébrale :
- empêche de faire jouer le cycle de satiété, d'autant plus que l'inflammation a déjà annihilé la régulation des hormones incrétines comme CCK et 6LP1,
- joue sur le sommeil, le raccourcissement du temps de sommeil pouvant ralentir la perte de poids et en stabilisation faire reprendre du poids,
- joue sur l'humeur, ce qui amplifie l'action du stress et peut conduire à la dépression.

La déficience en tryptophane provoque également des boulimies et des compulsions sucrées.

Or, les compulsions sucrées et la dépression font perdre moins de poids en phase active, et conduisent à une reprise de poids ensuite à long terme.

Les compulsions sucrées poussent la personne à remplacer les protéines et les glucides à charge glycémique basse par des sucres rapides à charges glycémique importante. La basse fréquence de la prise de protéines, en particulier de protéines animales, durant la période de perte de poids très rapide qui suit l'intervention, accélère une perte importante de la masse maigre musculaire, osseuse et des organes comme le coeur. Cette perte de masse maigre trop importante est le gage d'une reprise de poids car le métabolisme de base ne cesse de baisser. Dans la seule étude clinique qui soit analysée à un mois après l'opération (Carey - Surgery Obesity) le groupe passe de 140,8 kg à 128,5 kg mais incluant une perte de masse maigre de 4,3 kg soit 35% de la perte de poids, ce qui induit une baisse anormale du métabolisme de base (DER) de 17%. Ceci a pour effet d'entrainer un ralentissement dans les semaines qui suivent du rythme de la perte de poids et surtout une reprise de poids au moment de la stabilisation et à plus long terme une sarcopénie et une ostéoporose.

Concernant les conséquences de l'intervention de chirurgie bariatrique, l'état cétonique provoqué par le jeune après l'opération, renforce encore plus la tendance à la boulimie et aux compulsions sucrées des personnes obèses, en provoquant le remplacement du glucose nécessaire au cerveau par le β-hydroxybutirate dont un des substrats est le tryptophane aggravant ainsi la déficience naturelle.

De plus, les personnes ayant subi une intervention bariatrique ne peuvent plus mener la même vie qu'avant l'opération et un stress chronique s'installe, d'autant plus facilement que la plupart étaient stressés chroniquement, provoquant le catabolisme du tryptophane par le cortisol.

Par ailleurs, après l'opération, la quantité de nourriture pouvant être ingérée par les personnes ayant subies une opération de chirurgie bariatrique est faible, et sur cette faible quantité, les opérés favorisent la prise de lipides et de glucides, en particulier de glucides à charges glycémiques élevées, au détriment des protides. En plus des compulsions sucrées liées au statut d'obèse de la personne, la faible prise de protéines est également due au fait que les personnes opérées ont une appétence modérée pour les protéines animales en raison des changements de goût provoqués par l'intervention chirurgicale. Il existe donc un déficit général en protéines, et en particulier en acides aminés branchés, auquel s'ajoute pour les opérés en chirurgie bariatrique malabsorptive, une mauvaise absorption et un mauvais métabolisme des protéines au niveau intestinal, qui découle de l'opération elle-même et entraine des carences graves en protéines. Ce déficit peut provoquer une asthénie généralisée et peut conduire à une sarcopénie.

De plus, après une intervention de chirurgie bariatrique, la personne obèse ne peut s'alimenter que sous forme liquide et fractionnée pendant plusieurs semaines. Or, l'alimentation liquide est difficile à maîtriser car elle est souvent vite absorbée sans déclencher les signaux de satiété et elle provoque une rapide vidange gastrique. La consistance de la préparation est importante car pour la digestibilité il faut des produits de petits poids moléculaires qui sont très légers dans la solution, et pour la satiété il faut une pate compacte sans augmenter l'osmolalité. Le choix particulier des composants selon l'invention permet avantageusement de résoudre cette équation difficile. Il est important que l'osmolalité du produit en solution de 300l d'eau, capacité maximale d'un estomac opéré, ne dépasse pas 600mOsmol/kg soit le double de l'osmolalité de la formule sanguine. Au-delà, des troubles digestifs en particulier de « dumping » syndrome et de diarrhée peuvent survenir.

En outre, la composition selon l'invention, grâce à l'action combinée et synergique de ses composants, permet de lutter contre les nombreuses perturbations et contraintes mentionnées qui, si elles ne sont pas traitées, conduisent à l'échec des effets de l'opération et jouent sur la santé des patients obèses opérés.

La composition peut être utilisée comme produit de nutrition médicale à administration orale notamment pour :
- accélérer la diminution du gras viscéral de la personne obèse en phase post opératoire d'une opération de chirurgie bariatrique,
- augmenter le taux relatif de la masse maigre par rapport au poids total de la personne obèse en phase post opératoire d'une opération de chirurgie bariatrique,
- prévenir des risques de complications post opératoires chez la personne obèse en phase post opératoire d'une opération de chirurgie bariatrique, ces complications étant :
   - de nature nutritionnelle comme le dumping syndrome ou l'hypoglycémie post prandiale ou les carences soit physiologique comme la perte trop rapide de masse maigre par rapport à la masse grasse qui entrainera une probable reprise de poids, et/ou
   - chirurgicales dues à une nutrition inadaptée comme les fistules, les abcès, les hyper dilatations, les oesophagites, les dysphagies ou les vomissements, et/ou
   - médicales comme la dépression post opératoire par manque de sérotonine ou excès de cortisol.

La composition peut être utilisée pour remplacer l'alimentation de la personne obèse pendant au moins 4 semaines immédiatement après l'opération de chirurgie bariatrique, puis pour apporter un complément spécifique pour enrichir l'alimentation pendant une période de quelques semaines en fonction de l'état clinique et biologique du patient.

Les composants particuliers présents dans l'isolat et le concentrat de lactosérum permettent à la fois d'accélérer la perte du gras viscéral, de maintenir sa masse maigre et de jouer sur la sensation de satiété. On sait que les protéines sont à privilégier dans l'alimentation des personnes obèses pour maintenir la masse maigre, mais le choix particulier des protéines et acides aminés de la composition selon l'invention permet également de cibler la perte de masse grasse et de mobiliser les graisses sous-cutanées qui sont difficilement attaquables. En outre la présence de calcium permet de maintenir la masse osseuse et présente un effet coupe-faim.

Selon l'invention, le lactosérum est la seule source qui permet d'avoir la quantité nécessaire d'acides aminés branchés indispensables à l'efficacité et l'application recherchée. En outre, l'isolat et le concentrat sont les seules formes présentant une biodisponibilité de 30 à 45 minutes permettant de couvrir une alimentation fractionnée en tranches de 3 heures sans sentiment de faim, avec de la satiété et sans « dumping » syndrome.

L'osmolalité de la composition, inférieure à 600 mOsmol/kg, préférentiellement située à environ 300mOsmol/kg pour les saveurs sucrées et environ 500mOsmol/kg pour les saveurs salées, permet une parfaite digestibilité.

Par ailleurs, le faible poids moléculaire de ces composés, permet une assimilation optimale dans la portion restreinte d'un système digestif fragilisé par l'opération chirurgicale surtout en dérivation biliopancréatique et by-pass. Avantageusement, la composition selon l'invention est tout à fait adaptée à une alimentation sous forme liquide en correspondance avec l'état général de la personne obèse récemment opérée.

L'isolat et le concentrat de lactosérum de la composition selon l'invention ont chacun un poids moléculaire compris de façon préférée entre 15 000 et 20 000 daltons.

L'isolat de lactosérum est préférentiellement fabriqué à partir de lait frais et de fromageries qui ne pasteurisent pas le lait pour éviter la destruction des bêta-lactoglobuline et alpha-lactalbumine, et qui extraient le lactosérum par ultrafiltration ou microfiltration (taille des filtres de 0,1*µ*m). L'isolat obtenu par échange d'ions est moins adapté en raison de sa faible teneur en bêta-lactoglobuline et alpha-lactalbumine. L'isolat contient moins de 1% de lactose et de matières grasses, et sa concentration en peptides est préférentiellement d'au moins 90% en poids de matière sèche.

Le concentrat de lactosérum est préférentiellement obtenu à partir d'un lactosérum de fromagerie sans pasteurisation, contenant des bêta-lactoglobuline, des alpha-lactalbumine, des glycomacropeptides et des biopeptides. La concentration en peptides du concentrat est préférentiellement d'au moins 80% en poids de matière sèche.

De façon préférée, le ratio en poids entre l'isolat et le concentrat de lactosérum est de 1,5 à 2,5 dans la composition. Une telle caractéristique favorise la perte de gras viscéral, le maintien de la masse maigre et osseuse et donne un bon indice de digestibilité qui est essentiel pour les estomacs traumatisés des personnes visées.

Selon un mode de réalisation particulièrement adapté, la composition selon l'invention, comprend :
- entre 7 et 14% d'isolat de lactosérum, et
- entre 15 et 20% de concentrat de lactosérum,

les pourcentages étant donnés en poids de matière sèche de la totalité des principes actifs présents de la composition. Le rapport du concentrat sur l'isolat est préférentiellement de 2 à ±10%.

La composition selon l'invention peut également contenir des éléments ajoutés librement, tels que des acides aminés, des vitamines et des minéraux, qui viennent s'ajouter aux constituants natifs de l'isolat et du concentrat de lactosérum.

Selon un mode de réalisation particulièrement adapté, la composition comprend des acides aminés ajoutés sous forme libre, qui permettent d'améliorer encore son efficacité. Ces acides aminés peuvent être des acides aminés obtenus par fermentation.

Les acides aminés présents dans la composition, provenant de l'isolat ou du concentrat ou ajoutés sous forme libre, sont au moins la leucine, l'arginine, la taurine et/ou la glutamine.

Lorsque l'arginine et la taurine sont présents, le rapport du poids d'arginine sur le poids de taurine est préférentiellement de 2,5 à ±20%.

La leucine éventuellement associée à l'arginine et éventuellement à la vitamine D joue un rôle en particulier sur le maintien de la masse maigre. En effet, la leucine entre dans la synthèse des protéines, donc dans la formation de la masse maigre et la présence d'arginine, permet d'accélérer et d'amplifier cette synthèse. L'arginine et la taurine permettent aussi de remettre en état de marche les hormones incrétines gênées par l'inflammation locale de l'intestin. Cette action est favorisée en présence de zinc.

La composition comprend également préférentiellement au moins un acide aminé choisi parmi le tryptophane, l'isoleucine, la valine, la phénylalanine ou la tyrosine. Lorsque le tryptophane est présent, il doit représenter entre 5 et 7%, préférentiellement environ 6,5% en poids des acides aminés neutres présents dans la composition, à savoir tryptophane, leucine, isoleucine, valine, phénylalanine et tyrosine. Cette proportion particulière permet d'assurer qu'une quantité adaptée de tryptophane puisse traverser la barrière hématoencéphalique pour être transformée dans le cerveau en sérotonine de façon notamment à agir sur la sensation de satiété en complément des actions de l'arginine et de la taurine sur les hormones incrétines. De plus, il sert à favoriser la gestion du stress et à restaurer un sommeil réparateur et suffisant pour équilibrer le poids.

En plus de l'isolat, du concentrat de lactosérum, et des acides aminés libres, le mélange de principes actifs de la composition selon l'invention peut également comprendre un ou plusieurs des éléments suivants : le calcium, le magnésium, la vitamine B6, la vitamine B9, la vitamine E, la vitamine D, de zinc et le chrome. De même, la composition peut comprendre un ou plusieurs éléments choisis parmi le potassium, le sodium le sélénium, le phosphore, le fer, le manganèse, le cuivre, l'iode et le molybdène, les vitamines A, C, B1,B2, B3, B5, B8, B12.

La composition peut aussi contenir des acides gras essentiels, notamment des omégas 3. Il s'agit très préférentiellement d'omégas 3 d'origine végétale, avec une proportion élevée d'EPA.

Ces constituants complémentaires permettent d'améliorer encore l'effet de la composition. En particulier, ils permettent de combler des déficiences et de lutter contre des complications et contraintes liées soit à l'obésité (notamment déficiences en magnésium, calcium, zinc, chrome, vitamine B6 et B9, vitamine D et oméga 3), soit à l'opération et à l'alimentation particulière imposée en phase postopératoire immédiate d'une intervention de chirurgie bariatrique.

La présence de vitamine B6 et de magnésium dans la composition permet d'améliorer la synthèse de sérotonine à partir de tryptophane dans le cerveau. La vitamine B6 et le magnésium permettent également de renforcer l'effet coupe-faim du tryptophane.

L'apport de vitamine B9, permet d'améliorer encore l'action de la leucine sur la synthèse des protéines et le maintien de la masse maigre.

La vitamine B1 permet de combler des éventuelles déficiences provoquées par des vomissements, fréquents dans ces interventions, susceptibles de provoquer des ennuis neurologiques graves.

Le chrome, permet d'éviter l'apparition de pics d'insuline post-prandiaux qui peuvent provoquer des accidents cardiovasculaires et des « dumping » syndromes précoces.

La vitamine D permet d'éviter une perte trop importante de la masse maigre. En effet, lors de la perte de poids, un manque de vitamine D peut entraîner une perte de la masse maigre et osseuse qui peut devenir dangereuse et provoquer de la sarcopénie et de l'ostéoporose avec des accidents de fractures spontanées quelques années après l'opération, surtout pour les femmes après la ménopause. L'apport de calcium permet de renforcer l'action sur l'os de la vitamine D et joue également un rôle de coupe-faim.

Les acides gras essentiels, en particulier les oméga-3, permettent de limiter la sécrétion de fibrinogène. Chez la personne obèse, la localisation de la masse grasse au niveau abdominal développe une inflammation chronique de bas grade qui peut entraîner un diabète ou une maladie cardiovasculaire qui fragilise le patient pendant la récupération. Après l'opération, la perte de gras viscéral est la priorité pour combattre l'inflammation et arrêter l'athérogenèse et le risque d'éclatement des plaques d'athérome par la CRP ou la formation du thrombus par le fibrinogène. La CRP peut baisser le plus souvent lors d'une diète à très basse calories, mais rarement le fibrinogène. L'apport d'oméga-3 dans la composition selon l'invention permet donc de faire baisser le fibrinogène, et donc de diminuer le risque lié à l'inflammation. Par ailleurs cet apport d'oméga 3 permet aussi de lutter contre la dépression et le stress chronique.

Le fer, associé à la vitamine C, peut permettre d'éviter les anémies qui touchent les personnes opérées.

Par ailleurs, la composition selon l'invention permet d'éviter des complications dues à l'intervention. En particulier, les vitamines A, B, C, E, le fer, le zinc, le sélénium et la glutamine aident à la cicatrisation. D'autre part, l'apport de vitamines B et d'oméga-3 permet de limiter le risque de thromboses.

Les différents constituants de la composition selon l'invention agissent donc en synergie pour procurer des effets surprenants particulièrement adaptés à la phase post-opératoire immédiate d'une intervention de chirurgie bariatrique. Selon un mode de réalisation particulièrement adapté, la composition selon l'invention est constituée par au moins :
- 12 à 20% d'acides aminés branchés,
- 3 à 8% d'acides aminés aromatiques,
- 1 à 2% de taurine,
- 3 à 8% d'arginine,
- 0,3 à 2% de calcium,
- 0,3 à 2% d'omégas 3,
- 0,3 à 2% de magnésium pour 46g de composition sans excipients,
- 0,2 à 0,8 mg de vitamine B6 pour 46g de composition sans excipients,
- 2 à 4 mg de zinc pour 46g de composition sans excipients,
- 1 à 3 *µ*g de vitamine D pour 46g de composition sans excipients,
- 5 à 15 *µ*g de chrome pour 46g de composition sans excipients,
- 30 à 50 *µ*g de vitamine B9 pour 46g de composition sans excipients,
- 2 à 4 mg de vitamine E pour 46g de composition sans excipients,

les pourcentages étant donnés en poids de matière sèche de la totalité des principes actifs présents de la composition (en dehors des éventuels excipients), une partie des constituants provenant de l'isolat et du concentrat de lactosérum, et le reste étant ajouté librement sous forme d'acides aminés, de vitamines et de minéraux.

Les acides aminés branchés de la composition (totalité des acides aminés branchés provenant de l'isolat ou du concentrat de lactosérum et ajoutés librement) sont préférentiellement constitués de :
- 50 à 60% de leucine,
- 20 à 25% d'isoleucine, et
- 20 à 25% de valine,
   les pourcentages étant donnés en poids de matière sèche de chacun des acides aminés par rapport au poids de matière sèche total des acides aminés branchés. Les acides aminés aromatiques de la composition (totalité des acides aminés aromatiques provenant de l'isolat ou du concentrat de lactosérum et ajoutés librement) sont préférentiellement constitués de :
   - 15 à 25% de tryptophane,
   - 35 à 45% de phénylalanine,
   - 35 à 45% de tyrosine,
      les pourcentages étant donnés en poids de matière sèche de chacun des acides aromatiques par rapport au poids de matière sèche total des acides aminés aromatiques.

La composition selon l'invention peut être obtenue par simple mélange des constituants.

Préférentiellement, elle est obtenue par la mise en oeuvre des étapes suivantes :
- introduire et mélanger dans l'ordre sous forme de poudre l'isolat de lactosérum et le concentrat de lactosérum, et éventuellement les acides aminés libres, le magnésium et le calcium.
- éventuellement ajouter au premier mélange sous forme de poudre les vitamines, les minéraux et les acides gras.

On obtient ainsi une poudre qui peut être transformée en comprimé ou liquide, ou bien utilisée dans sa forme poudre en sachets, sticks, bidons ou gélules par exemple.

La composition selon l'invention peut se présenter sous toute forme adaptée à une administration par voie orale. Elle peut notamment se présenter sous forme de poudre ou granulés, de boissons prêtes à l'emploi, de barres ou d'extrudés, la composition étant additionnée d'excipients et charges classiques connues de l'homme du métier.

Préférentiellement, elle se présente sous forme de poudre ou granules conditionnée dans un sachet à diluer dans l'eau.

La dose journalière de composition selon l'invention (dose de mélange de principes actifs sans les excipients) est préférentiellement comprise entre 175 et 250g, de préférence en cinq prises de 35 à 50g réparties toutes les 3 heures. Avantageusement, la biodisponibilité dans l'organisme des acides aminés, peptides et protéines présents dans la composition dans l'organisme est comprise entre 0 et 2 heures, ce qui permet une action à la fois rapide et qui perdure dans le temps de façon à limiter la quantité de nourriture.

Selon un autre avantage, la composition selon l'invention permet d'éviter le syndrome de dumping précoce (vidange gastrique rapide). Ce syndrome qui touche un patient opéré en chirurgie bariatrique sur deux est provoqué par la composition de la nourriture en ce qui concerne la taille des molécules de protéines ainsi que l'osmolarité de l'ensemble du bol alimentaire qui est réduit en quantité entraînant une augmentation significative de la concentration sur une faible quantité de liquide. Les protéines supérieures à 50 000 daltons ne sont pas totalement digestes et provoquent des gênes comme les vomissements et un rejet ultérieur des protéines. De même, les omégas 3 d'origine animale en raison de leur relent de poisson qui entraîne un abandon du produit. Le choix des protéines et des omégas présents dans la composition selon l'invention permet d'éviter ces problématiques. De plus, le choix particulier des proportions, quantités et qualité des protéines présentes dans la composition selon l'invention permet d'avoir une osmolalité la plus basse possible (inférieure à 600 mOsm/kg) de façon à éviter des malaises et le syndrome de dumping gastrique causés par une hyper osmolarité associée à une grande quantité de glucose.

L'invention est à présent illustrée par un exemple non limitatif de composition diététique, se présentant sous forme d'une poudre de 50 g (principes actifs et excipients) conditionnée dans un sachet.

Cette composition est obtenue à partir des principes actifs suivants :
- 3,7 g d'isolat de lactosérum de poids moléculaire entre 15000 et 20000 daltons,
- 7,8 g de concentrat de lactosérum de poids moléculaire entre 15000 et 20000 daltons,
- 0,28 mg de vitamine B6,
- 2,0 mg de zinc,
- 0,6 g de taurine,
- 8 *µ*g de chrome,
- 2,4 mg de vitamine E,
- 40 *µ*g de vitamine B9,
- 1 *µ*g de vitamine D,
- 200 mg d'omégas 3 d'origine végétale,
- qsp 4g de leucine,
- qsp 0.5g de tryptophane,
- qsp 1.6 g d'arginine,
- qsp 0,16g de calcium,
- qsp 75 mg de magnésium.

Par « qsp *Xg* » d'un élément de la composition, on entend la quantité totale de cet élément dans la composition : quantité apportée par les protides (isolat de lactosérum, concentrat de lactosérum) et complétée par un ajout de l'élément sous forme libre pour arriver jusqu'à *X*g.

## Revendications

1. Composition diététique comprenant au moins les constituants suivants :
- un isolat de lactosérum, et
- un concentrat de lactosérum,
l'isolat de lactosérum ayant un poids moléculaire compris entre 15000 et 20000 daltons et/ou le concentrat de lactosérum ayant un poids moléculaire compris entre 15000 et 20000 daltons, et le ratio en poids entre l'isolat et le concentrat de lactosérum étant de 1,5 à 2,5, pour son utilisation comme produit de nutrition médicale à administration orale pour améliorer l'état médical d'une personne obèse en phase post opératoire d'une opération de chirurgie bariatrique, à savoir :
- pour faire accélérer la diminution du gras viscéral et augmenter le rapport de la masse maigre sur le poids total de la personne obèse en phase post opératoire, et
- pour prévenir des risques de complications post opératoires choisies parmi le dumping syndrome, l'hypoglycémie post prandiale, les carences physiologiques, les fistules, les abcès, les hyper dilatations, les oesophagites, les dysphagies, les vomissements et la dépression post opératoire.

2. Composition selon la revendication 1, pour son utilisation comme produit de nutrition médicale à administration orale pour remplacer l'alimentation de la personne obèse pendant au moins 4 semaines immédiatement après l'opération de chirurgie bariatrique.

3. Composition selon l'une des précédentes revendications, **caractérisée en ce qu'**elle comprend également un ou plusieurs acides aminés.

4. Composition selon l'une des précédentes revendications, **caractérisée en ce qu'**elle comprend au moins un des acides aminés choisis parmi la leucine, l'arginine, la taurine et /ou la glutamine.

5. Composition selon la revendication 3 ou 4 , **caractérisée en ce qu'**elle comprend de la leucine et **en ce que** la leucine représente 50 à 60% en poids des acides aminés branchés présents dans la composition.

6. Composition selon la revendication 4 ou 5, **caractérisée en ce que** le rapport du poids d'arginine sur le poids de taurine est de 2,5 ± 20%.

7. Composition selon l'une des revendications 4 à 6, **caractérisée en ce qu'**elle comprend également au moins un acide aminé choisi parmi le tryptophane, l'isoleucine, la valine, la phénylalanine et la tyrosine.

8. Composition selon la revendication 7, caractérisée en que le tryptophane représente entre 5 et 7% en poids des acides aminés neutres présents dans la composition.

9. Composition selon l'une des précédentes revendications, **caractérisée en ce que** le mélange de principes actifs comprend également au moins un des éléments choisis parmi le calcium, le magnésium, la vitamine B6, la vitamine B9, la vitamine E, la vitamine D, le zinc et le chrome.

10. Composition selon l'une des précédentes revendications, **caractérisée en ce que** le mélange de principes actifs comprend également au moins un des éléments choisis parmi le potassium, le sodium, le sélénium, le phosphore, le fer, le manganèse, le cuivre, l'iode, le molybdène, les vitamines A, C, B1, B2, B5, B8, B12.

11. Composition selon l'une des précédentes revendications, **caractérisée en ce que** le mélange de principes actifs comprend également des acides gras essentiels.

12. Composition selon la revendication 16, **caractérisée en ce que** les acides gras essentiels sont des omégas 3.

13. Composition selon l'une des précédentes revendications, **caractérisée en ce que** l'isolat de lactosérum représente entre 7 et 14%, et le concentrat de lactosérum entre 15 et 20%, les pourcentages étant donnés en poids de matière sèche de la totalité des principes actifs présents de la composition.

14. Composition selon l'une des précédentes revendications, **caractérisée en ce qu'**elle est constituée par au moins :
- 12 à 20% d'acides aminés branchés,
- 3 à 8% d'acides aminés aromatiques,
- 1 à 2% de taurine,
- 3 à 8% d'arginine,
- 0,3 à 2% de calcium,
- 0,3 à 2% d'omégas 3,
- 0,3 à 2% de magnésium pour 46g de composition sans excipients,
- 0,2 à 0,8 mg de vitamine B6 pour 46g de composition sans excipients,
- 2 à 4 mg de zinc pour 46g de composition sans excipients,
- 1 à 3 µg de vitamine D pour 46g de composition sans excipients,
- 5 à 15 µg de chrome pour 46g de composition sans excipients,
- 30 à 50 µg de vitamine B9 pour 46g de composition sans excipients,
- 2 à 4 mg de vitamine E pour 46g de composition sans excipients, les pourcentages étant donnés en poids de matière sèche de la totalité des principes actifs présents de la composition.

15. Composition selon l'une des précédentes revendications, **caractérisée en ce que** son osmolalité en solution de 300l d'eau ne dépasse pas 600mOsm/kg.

16. Composition selon l'une des précédentes revendications, **caractérisée en ce qu'**elle se présente sous forme de poudre ou granulés, de boisson prête à l'emploi, de barres alimentaires ou extrudés.

17. Composition selon l'une des précédentes revendications, pour son utilisation comme produit de nutrition médicale à administration orale à raison de cinq doses de 35 à 50g de la totalité des principes actifs présents dans la composition par jour.

## Patentansprüche

1. Diätetische Zusammensetzung, die wenigstens die folgenden Bestandteile aufweist:
- ein Isolat aus Milchserum, und
- ein Konzentrat aus Milchserum, wobei das Isolat aus Milchserum ein Molekulargewicht zwischen 15.000 und 20.000 Daltons und/oder das Konzentrat aus Milchserum ein Molekulargewicht zwischen 15.000 und 20.000 Daltons aufweist und das Gewichtsverhältnis zwischen dem Isolat und dem Konzentrat aus Milchserum zwischen 1,5 und 2,5 liegt, für deren Verwendung als medizinisches Nahrungsmittel für die orale Anwendung zur Verbesserung des medizinischen Zustands einer adipösen Person in der postoperativen Phase einer Operation der bariatrischen Chirurgie, nämlich:
- um die Verringerung des viszeralen Fettgewebes zu beschleunigen und das Verhältnis des mageren Gewebes bezüglich des Gesamtgewichts der adipösen Person in der postoperativen Phase zu erhöhen, und
- um dem Risiko postoperativer Komplikationen vorzubeugen, die aus dem Dumpingsyndrom, der Hypoglykämie nach Mahlzeiten, physiologischen Mangelerscheinungen, Fisteln, Abszessen, Hyperdilatationen, den Speiseröhrenentzündungen, den Schluckstörungen, den Erbrechungsvorgängen und der postoperativen Depression ausgewählt sind.

2. Zusammensetzung nach Anspruch 1, für deren Verwendung als medizinisches Ernährungsprodukt zur oralen Anwendung, um die Ernährung der adipösen Person während wenigstens vier Wochen unmittelbar nach der Operation der bariatrischen Chirurgie zu ersetzen.

3. Zusammensetzung nach einem der vorhergehenden Ansprüche, **dadurch gekennzeichnet, dass** diese auch eine oder mehrere Aminosäuren aufweist.

4. Zusammensetzung nach einem der vorhergehenden Ansprüche, **dadurch gekennzeichnet, dass** diese wenigstens eine Aminosäure aufweist, die unter Leucin, Arginin, Taurin und/oder Glutamin ausgewählt sind.

5. Zusammensetzung nach Anspruch 3 oder 4, **dadurch gekennzeichnet, dass** diese Leucin aufweist, und dass Leucin 50 bis 60 Gew.-% der verzweigten Aminosäuren darstellt, die in der Zusammensetzung vorhanden sind.

6. Zusammensetzung nach Anspruch 4 oder 5, **dadurch gekennzeichnet, dass** das Verhältnis des Gewichts von Arginin zum Gewicht von Taurin im Bereich von 2,5 ± 20% liegt.

7. Zusammensetzung nach einem der Ansprüche 4 bis 6, **dadurch gekennzeichnet, dass** diese auch wenigstens eine Aminosäure aufweist, die unter Tryptophan, Isoleucin, Valin, Phenylalanin und Tyrosin ausgewählt ist.

8. Zusammensetzung nach Anspruch 7, **dadurch gekennzeichnet, dass** Tryptophan zwischen 5 und 7 Gew.-% der in der Zusammensetzung vorhandenen neutralen Aminosäuren darstellt.

9. Zusammensetzung nach einem der vorhergehenden Ansprüche, **dadurch gekennzeichnet, dass** die Mischung aus Wirkstoffen auch wenigstens einen der Bestandteil aufweist, die aus Kalzium, Magnesium, Vitamin B6, Vitamin B9, Vitamin E, Vitamin D, Zink und Chrom ausgewählt sind.

10. Zusammensetzung nach einem der vorhergehenden Ansprüche, **dadurch gekennzeichnet, dass** die Mischung aus Wirkstoffen auch wenigstens eines der Elemente aufweist, die unter Kalium, Natrium, Selen, Phosphor, Eisen, Mangan, Kupfer, Iod, Molybdän und den Vitaminen A, C, B1, B2, B5, B8, B12 ausgewählt sind.

11. Zusammensetzung nach einem der vorhergehenden Ansprüche, **dadurch gekennzeichnet, dass** die Mischung aus Wirkstoffen auch essentielle Fettsäuren aufweist.

12. Zusammensetzung nach Anspruch 16, **dadurch gekennzeichnet, dass** die essentiellen Fettsäuren Omega-3-Fettsäuren sind.

13. Zusammensetzung nach einem der vorhergehenden Ansprüche, **dadurch gekennzeichnet, dass** das Isolat aus Milchserum zwischen 7 und 14% und das Konzentrat aus Milchserum zwischen 15 und 20% darstellt, wobei die Prozentangaben auf das Trockengewicht der gesamten, in der Zusammensetzung vorhandenen Wirkstoffe bezogen sind.

14. Zusammensetzung nach einem der vorhergehenden Ansprüche, **dadurch gekennzeichnet, dass** diese sich wenigstens zusammensetzt aus:
- 12 bis 20% verzweigte Aminosäuren,
- 3 bis 8% aromatische Aminosäuren,
- 1 bis 2% Taurin,
- 3 bis 8% Arginin,
- 0,3 bis 2% Kalzium,
- 0,3 bis 2% Omega-3-Fettsäuren,
- 0,3 bis 2% Magnesium je 46 g Zusammensetzung ohne Hilfsstoffe,
- 0,2 bis 0,8 mg Vitamin B6 je 46 g Zusammensetzung ohne Hilfsstoffe,
- 2 bis 4 mg Zink je 46 g Zusammensetzung ohne Hilfsstoffe,
- 1 bis 3 µg Vitamin D je 46 g Zusammensetzung ohne Hilfsstoffe,
- 5 bis 15 µg Chrom je 46 g Zusammensetzung ohne Hilfsstoffe,
- 30 bis 50 µg Vitamin B9 je 46 g Zusammensetzung ohne Hilfsstoffe,
- 2 bis 4 mg Vitamin E je 46 g Zusammensetzung ohne Hilfsstoffe,
wobei die Prozentangaben auf das Trockengewicht der gesamten, in der Zusammensetzung vorhandenen Wirkstoffe bezogen sind.

15. Zusammensetzung nach einem der vorhergehenden Ansprüche, **dadurch gekennzeichnet, dass** deren Osmolalität in Lösung mit 300 l Wasser nicht 600 mOsm/kg übersteigt.

16. Zusammensetzung nach einem der vorhergehenden Ansprüche, **dadurch gekennzeichnet, dass** diese als Pulver oder Granulat, als Fertiggetränk, als Nahrungsriegel oder stranggepresster Riegel ausgebildet ist.

17. Zusammensetzung nach einem der vorhergehenden Ansprüche, für deren Verwendung als medizinisches Ernährungsprodukt für die orale Anwendung mit fünf Dosen je 35 bis 50 g der insgesamt in der Zusammensetzung vorhandenen Wirkstoffe pro Tag.

## Claims

1. A dietetic composition comprising at least the following constituents:
- a lactoserum isolate, and
- a lactoserum concentrate,
the lactoserum isolate having a molecular weight of between 15,000 and 20,000 daltons and/or the lactoserum concentrate having a molecular weight of between 15,000 and 20,000 daltons, the ratio by weight between the lactoserum isolate and concentrate being 1.5 to 2.5, for use thereof as a medical nutrition product for oral administration in order to improve the medical state of an obese person in the post-operative phase of a bariatric surgery operation, namely:
- to accelerate the reduction in visceral fat and to increase the ratio of lean mass to the total weight of the obese person in the post-operative phase, and
- to prevent risks of post-operative complications chosen from dumping syndrome, post prandial hypoglycaemia, physiological deficiencies, fistulas, abscesses, hyperdilations, oesophagitis, dysphagia, vomiting and post-operative depression.

2. A composition according to claim 1, for use thereof as a medical nutrition product for oral administration for replacing the food of the obese person for at least four weeks immediately after the bariatric surgery operation.

3. A composition according to one of the preceding claims, **characterised in that** it also comprises one or more amino acids.

4. A composition according to one of the preceding claims, **characterised in that** it comprises at least one of the amino acids chosen from leucine, arginine, taurine and/or glutamine.

5. A composition according to claim 3 or 4, **characterised in that** it comprises leucine and **in that** the leucine represents 50% to 60% by weight of the branched amino acids present in the composition.

6. A composition according to claim 4 or 5, **characterised in that** the ratio of the weight of arginine to the weight of taurine is 2.5 ± 20%.

7. A composition according to one of claims 4 to 6, **characterised in that** it also comprises at least one amino acid chosen from tryptophan, isoleucine, valine, phenylalanine and tyrosine.

8. A composition according to claim 7, **characterised in that** the tryptophan represents between 5% and 7& by weight of the neutral amino acids present in the composition.

9. A composition according to one of the preceding claims, **characterised in that** the mixture of active principles also comprises at least one of the substances chosen from calcium, magnesium, vitamin B6, vitamin B9, vitamin E, vitamin D, zinc and chromium.

10. A composition according to one of the preceding claims, **characterised in that** the mixture of active principles also comprises at least one of the substances chosen from potassium, sodium, selenium, phosphorus, iron, manganese, copper, iodine, molybdenum and vitamins A, C, B1, B2, B5, B8 and B12.

11. A composition according to one of the preceding claims, **characterised in that** the mixture of active principles also comprises essential fatty acids.

12. A composition according to claim 16, **characterised in that** the essential fatty acids are omega-3 fatty acids.

13. A composition according to one of the preceding claims, **characterised in that** the lactoserum isolate represents between 7% and 14%, and the lactoserum concentrate between 15% and 20%, the percentages being given by weight of dry matter of all the active principles present in the composition.

14. A composition according to one of the preceding claims, **characterised in that** it comprises at least:
- 12% to 20% branched amino acids,
- 3% to 8% aromatic amino acids,
- 1% to 2% taurine,
- 3% to 8% arginine,
- 0.3% to 2% calcium,
- 0.3% to 2% omega 3,
- 0.3% to 2% magnesium for 46 g of composition without excipients,
- 0.2 to 0.8 mg of vitamin B4 for 46 g of composition without excipients,
- 2 to 4 mg of zinc for 46 g of composition without excipients,
- 1 to 3 µg of vitamin D for 46 g of composition without excipients,
- 5 to 15 µg of chromium for 46 g of composition without excipients,
- 30 to 50 µg of vitamin B9 for 46 g of composition without excipients,
- 2 to 4 mg of vitamin E for 46 g of composition without excipients,
the percentages being given by weight of dry matter of all the active principles present in the composition.

15. A composition according to one of the preceding claims, **characterised in that** its osmolality in solution of 300 litres of water does not exceed 600 mOsm/kg.

16. A composition according to one of the preceding claims, **characterised in that** it is in the form of powder or granules, a beverage ready for use, food bars or extrusions.

17. A composition according to one of the preceding claims, for use thereof as a medical nutrition product for oral administration at the rate of five doses of 35 g to 50 g of all the active principles present in the composition per day.
